# EUROPÄISCHE PATENTSCHRIFT

(11) **EP 4 180 028 B1**
(45) Veröffentlichungstag und Bekanntmachung des Hinweises auf die Patenterteilung: **23.10.2024**
(21) Anmeldenummer: 21208128.5
(22) Anmeldetag: 15.11.2021
(51) Int. Cl.: A61K 8/14, A61K 8/55, A61K 8/60, A61K 8/73, A61K 8/9789, A61Q 5/06

(54) **HAARFÄRBEMITTEL**
HAIR COLOURANT
COLORANT POUR CHEVEUX

(43) Veröffentlichungstag der Anmeldung: 17.05.2023
(73) Patentinhaber: Be Organic Natural Certified Products GmbH & Co. KG, 65183 Wiesbaden (DE)
(72) Erfinder: KRAHN, Bahar, 65183 Wiesbaden (DE)
(74) Vertreter: Richardt Patentanwälte PartG mbB

(56) Entgegenhaltungen:
- EP-A1- 1 800 652
- CN-A- 109 806 212
- DE-A1- 3 207 037
- FILOMENAMEHENDI: "Tutorial - Cassia and henna paste recipe for hair by Filomena's Mehendi", 16 October 2019 (2019-10-16), XP055910385, Retrieved from the Internet <URL:https://www.youtube.com/watch?v=FTO-7q29EVI> [retrieved on 20220407]
- ANONYMOUS: "The Henna Page - The Encyclopedia of Henna ~ Sugar and Henna", 5 March 2006 (2006-03-05), XP055910352, Retrieved from the Internet <URL:https://web.archive.org/web/20060305035658/http://www.hennapage.com/henna/encyclopedia/sugars/> [retrieved on 20220407]
- ANONYMOUS: "Henna Blog Spot > Common Mistakes and Tips: Henna for Hair", 18 August 2010 (2010-08-18), XP055910346, Retrieved from the Internet <URL:https://web.archive.org/web/20100818161636/http://hennablogspot.com/common-mistakes-and-tips-henna-for-hair> [retrieved on 20220407]

## Beschreibung

Die vorliegende Erfindung betrifft ein Haarfärbemittel, insbesondere ein temporäres Haarfärbemittel auf Basis natürlicher Inhaltsstoffe.

Eine Haarfärbung wird vorgenommen, um dem Haar andere Farbnuancen zu geben, das heißt die natürliche Haarfarbe zu verändern. Eine Haarfärbung ist meist auch noch nach mehrmaliger Haarwäsche mit tensidhaltigen Waschmitteln farbecht.

Farbänderungen des Haares kann man durch Blondiermittel oder Haarfärbemittel erreichen. Die Haarfärbung lässt sich in Bezug auf die Zahl der Haarwäschen nach der Färbung und der jeweiligen Haltbarkeit der Farbänderung in temporäre, semipermanente und permanente Färbungen einteilen. Eine temporäre Haarfärbung verschwindet nach dem ersten Waschen mit einem Haarshampoo. Sie basiert auf einer physikalischen Haftung eines Farbstoffes. Eine semipermanente Haarfärbung hält circa zwei bis zehn Haarwäschen aus. Eine permanente Färbung übersteht mehr als zehn Haarwäschen. Hierbei werden kosmetische Farbstoffe chemisch an bestimmte Aminosäuren im Haar gekoppelt oder beim Blondieren Pigmente des Haares oxidiert.

Viele herkömmliche Haarfärbemittel enthalten gesundheitlich problematische Inhaltsstoffe, die beispielsweise über die Haut, insbesondere die Kopfhaut, in den Körper aufgenommen werden und zu negativen Nebenwirkungen führen können.

CN109806212 A offenbart einen natürlichen Haarfärbemittel enthaltend ein natürliches Pigment, ein Liposom (Tensid wie Lecithin) und ein Polysaccharid-enthaltenden Zusammensetzung (Verdicker wie Xanthan gum, Gummi arabicum). DE3207037 A1 offenbart ein pulverförmiges Haarfärbemittel enthaltend natürliche Pigmente wie Hennablätter, Kamillenblüte, Sandelholz usw. und Verdünnungsmittel wie Zucker.

Wünschenswert sind daher Haarfärbemittel auf Basis natürlicher Inhaltsstoffe, die derartige negative Nebenwirkungen möglichst vermeiden. Herkömmliche Haarfärbemittel auf Basis natürlicher Pigmente umfassen jedoch häufig qualitativ minderwertige Inhaltsstoffe, sind oftmals auch nicht zertifiziert, haben eine unbefriedigende und unzureichende Farbwirkung und Deckkraft und sind in der Anwendung sowie bei der Handhabung meist unzureichend. Diese Probleme schrecken Anwender häufig ab. So sind insbesondere Schwierigkeiten bei Zweitfärbungen, zu lange Einwirkzeiten, schlechte Konsistenz des Haarfärbemittels sowie ein nicht einheitliches Farbbild der resultierenden Färbung zu beklagen, die oftmals zum Rückgriff auf Haarfärbemittel auf Basis nicht natürlicher, sondern chemischer Inhaltsstoffe, führen, wobei die zuvor diskutierten gesundheitlichen Probleme als kleineres Übel bewusst in Kauf genommen werden.

Aufgabe der vorliegenden Erfindung war die Bereitstellung eines Haarfärbemittels auf Basis natürlicher Inhaltsstoffe mit besonders vorteilhaften Eigenschaften, welches die Haarfärbung auf möglich einfache Art und Weise und möglichst kostengünstig ermöglicht. Gewünscht wurden insbesondere eine möglichst gute Konsistenz der Textur und Streichfähigkeit des gefärbten Haares, eine möglichst gute Verarbeitung der fertigen Textur der gefärbten Haares, ein möglichst gleichmäßiges Färbeergebnis durch die Haarfärbung, eine möglichst hohe Deckkraft der Farbe, eine möglichst gute Grauhaarabdeckung unter Verwendung der Haarfärbung, eine möglichst geringe Wasserabsonderung aus der Textur der Haarfärbung heraus, eine möglichst gute Glanz- und Pflegewirkung unter Verwendung der Haarfärbung, ein möglichst großes Volumen bei Verwendung der Haarfärbung, eine möglichst gute Haarstruktur nach Anwendung des Haarfärbemittels, eine möglichst gute Auswaschbarkeit und Entfernbarkeit der Haarfärbung aus dem Haar und ein möglichst geringer Wasserbedarf zur Anwendung der Haarfärbung.

Gleichzeitig wurde ein Haarfärbemittel auf Basis natürlicher Inhaltsstoffe gewünscht, um mögliche gesundheitliche Probleme bestmöglich zu vermeiden.

Gelöst werden diese Aufgaben der vorliegenden Erfindung durch die Bereitstellung eines Haarfärbemittels mit allen Merkmalen des vorliegenden Patentanspruchs 1. Die auf Patentanspruch 1 rückbezogenen Unteransprüche beschreiben besonders vorteilhafte Ausgestaltungen des erfindungsgemäßen Haarfärbemittels. Weiterhin wird ein besonders vorteilhaftes Verfahren zur Herstellung des erfindungsgemäßen Haarfärbemittels unter Schutz gestellt.

Durch die Bereitstellung eines Haarfärbemittels, umfassend, vorzugsweise jeweils bezogen auf 100 Gewichtsteile Haarfärbemittel,
1,0 bis 90,0 Gewichtsteile mindestens einer Zusammensetzung, die mindestens ein natürliches Pigment umfasst,
5,0 bis 35,0 Gewichtsteile mindestens eines Zuckers,
1,0 bis 10,0 Gewichtsteile mindestens eines Liposoms,
1,0 bis 8,0 Gewichtsteile einer Polysacharid-enthaltenden Zusammensetzung,
gelingt es auf überraschende Art und Weise, ein Haarfärbemittel mit besonders vorteilhaften Eigenschaften zugänglich zu machen, welches die Haarfärbung auf vergleichbar einfache Art und Weise und überaus kostengünstig ermöglicht. Erzielt werden insbesondere eine sehr gute Konsistenz der Textur und Streichfähigkeit des gefärbten Haares, eine sehr gute Verarbeitung der fertigen Textur des gefärbten Haares, ein sehr gutes und gleichmäßiges Färbeergebnis durch die Haarfärbung, eine sehr gute Deckkraft der Farbe, eine sehr gute Grauhaarabdeckung unter Verwendung der erfindungsgemäßen Haarfärbung, eine sehr geringe Wasserabsonderung aus der Textur der Haarfärbung heraus, eine sehr gute Glanz- und Pflegewirkung unter Verwendung der erfindungsgemäßen Haarfärbung, ein sehr großes Volumen bei Verwendung der erfindungsgemäßen Haarfärbung, eine sehr gute Haarstruktur, eine sehr gute Auswaschbarkeit und Entfernbarkeit der erfindungsgemäßen Haarfärbung aus dem Haar. Gleichzeitig ist der Wasserbedarf zur Anwendung der erfindungsgemäßen Haarfärbung sehr gering.

Auch eine Entmischung der Haarfarbe während ihrer Aufbringung, bei welcher sich Wasser im Laufe der Einwirkzeit von den übrigen Inhaltsstoffen trennt und Kleidung, Körperstellen, insbesondere Hautpartien im Gesicht, Ohren, Nacken, verschmutzt, wird nicht beobachtet. Im Gegenteil wird erfindungsgemäß ein nie dagewesener Anwendungskomfort sowie ein brillantes Farbergebnis beobachtet.

Gleichzeitig werden durch die Bereitstellung eines Haarfärbemittels auf Basis natürlicher Inhaltsstoffe mögliche gesundheitliche Probleme bestmöglich zu vermieden.

Haare bezeichnen erfindungsgemäß vorzugsweise lange Hornfäden, die auf der Haut von Säugetieren wachsen oder gewachsen sind. Haare bestehen im Wesentlichen aus Keratin. Bevorzugt handelt es sich um Kopfhaar, besonders bevorzugt um Haupthaar, Barthaar oder Augenbrauen, insbesondere um Kopfhaar.

Das erfindungsgemäße Haarfärbemittel umfasst, vorzugsweise bezogen auf 100 Gewichtsteile Haarfärbemittel, 1,0 bis 90,0 Gewichtsteile, bevorzugt 5,0 bis 75,0 Gewichtsteile, ganz besonders 25,0 bis 70,0 Gewichtsteile, insbesondere 50,0 bis 65,0 Gewichtsteile, einer Zusammensetzung, die mindestens ein natürliches Pigment (im Folgenden gelegentlich als Naturfarbstoff bezeichnet) umfasst.

Pigmente sind Farbmittel, also farbgebende Substanzen. Im Gegensatz zu Farbstoffen sind sie im Anwendungsmedium praktisch unlöslich und liegen dort als Feststoff-Teilchen vor.

Der Farbreiz entsteht durch Absorption und Remission (Streuung oder Reflexion) bestimmter Frequenzanteile des sichtbaren Lichts. Maßgeblich für die Eigenschaften der Pigmente sind Festkörpereigenschaften wie Kristallstruktur, Kristallmodifikation, Teilchengröße und Teilchengrößenverteilung, letztere durch die spezifische Oberfläche.

Für die Zwecke der vorliegenden Erfindung besonders geeignete natürliche Pigmente sind alle in Pflanzen, Tieren und Mikroorganismen enthaltenen und aus diesen gewinnbaren Farbstoffe, insbesondere solche, die bereits zur Färbung von Keratinfasern oder von Haaren verwendet worden sind. Eine Übersicht über natürliche Farbstoffe ist aus Ullmanns Encyclopädie der technischen Chemie, 4. Auflage (19), Band 11, Seite 100 bis 134 zu entnehmen.

Im Rahmen der vorliegenden Erfindung hat es sich als vorteilhaft erwiesen, wenn der Naturfarbstoff ausgewählt ist aus Henna rot, Henna neutral, Henna schwarz, Kamillenblüte, Sandelholz, schwarzem Tee, grünem Tee, Faulbaumrinde, Salbei, Blauholz, Krappwurzel, Catechu, Sedre, Alkannawurzel, Kurkumawurzel, Walnussschale, Walnussblätter, Sennesblätter, Indigo und Cochenille, wobei sich die Verwendung von Henna, Kurkumawurzel, Walnussschale, Walnussblätter, Sennesblätter und/oder Faulbaumrinde ganz besonders bewährt hat.

Der Naturstoff kann grundsätzlich in beliebiger Form eingesetzt werden. Besonders bewährt haben sich jedoch zerkleinerte, insbesondere gemahlene Formen sowie Extrakte der Naturstoffe.

Das erfindungsgemäße Haarfärbemittel enthält die Naturfarbstoffe bevorzugt in einer Menge von 0,01 bis 20 Gewichts-%, bezogen auf das Haarfärbemittel.

Vorzugsweise umfasst das erfindungsgemäße Haarfärbemittel, zweckmäßigerweise bezogen auf 100 Gewichtsteile Haarfärbemittel, 10,0 bis 40,0 Gewichtsteile, bevorzugt 15,0 bis 35,0 Gewichtsteile insbesondere 20,0 bis 30,0 Gewichtsteile bevorzugt zerkleinerte Walnussschalen.

Vorzugsweise umfasst das erfindungsgemäße Haarfärbemittel, zweckmäßigerweise bezogen auf 100 Gewichtsteile Haarfärbemittel, 8,0 bis 38,0 Gewichtsteile, bevorzugt 13,0 bis 33,0 Gewichtsteile insbesondere 18,0 bis 28,0 Gewichtsteile Henna.

Vorzugsweise umfasst das erfindungsgemäße Haarfärbemittel, zweckmäßigerweise bezogen auf 100 Gewichtsteile Haarfärbemittel, 1,0 bis 20,0 Gewichtsteile, bevorzugt 3,0 bis 16,0 Gewichtsteile insbesondere 4,0 bis 12,0 Gewichtsteile bevorzugt zerkleinerte Walnussblätter.

Vorzugsweise umfasst das erfindungsgemäße Haarfärbemittel, zweckmäßigerweise bezogen auf 100 Gewichtsteile Haarfärbemittel, 0,1 bis 10,0 Gewichtsteile, bevorzugt 0,5 bis 5,0 Gewichtsteile insbesondere 1,0 bis 3,0 Gewichtsteile bevorzugt zerkleinerte Faulbaumrinde.

Vorzugsweise umfasst das erfindungsgemäße Haarfärbemittel, zweckmäßigerweise bezogen auf 100 Gewichtsteile Haarfärbemittel, 0,1 bis 4,0 Gewichtsteile, bevorzugt 0,3 bis 2,5 Gewichtsteile insbesondere 0,5 bis 1,5 Gewichtsteile bevorzugt zerkleinerte Kurkumawurzel.

Vorzugsweise umfasst das erfindungsgemäße Haarfärbemittel, zweckmäßigerweise bezogen auf 100 Gewichtsteile Haarfärbemittel, 0,1 bis 4,0 Gewichtsteile, bevorzugt 0,3 bis 2,5 Gewichtsteile insbesondere 0,5 bis 1,5 Gewichtsteile bevorzugt zerkleinerte Sennesblätter.

Weiterhin umfasst das erfindungsgemäße Haarfärbemittel, vorzugsweise bezogen auf 100 Gewichtsteile Haarfärbemittel, 5,0 bis 35,0 Gewichtsteile, bevorzugt 7,0 bis 30,0 Gewichtsteile, ganz besonders 8,0 bis 25,0 Gewichtsteile, insbesondere 10,0 bis 25,0 Gewichtsteile, mindestens eines Zuckers.

Sofern die genannten Mengen unterschritten werden, können sich die technologiestützenden Eigenschaften des Zuckers, respektive der Farbe nicht optimal entwickeln. Der gewünschte Effekt bleibt aus. Eine größere Menge an Zucker verdünnt wiederum die Farbe sehr stark, da die Restbestandteile des Haarfärbemittels nicht den gewünschten Farbeffekt erzielen können, da die färbenden Stoffe zu niedrig dosiert wären.

Im Rahmen der vorliegenden Erfindung bindet der Zucker Feuchtigkeit im erfindungsgemäßen Haarfärbemittel und verhindert so das Austrocken von anderen Bestandteilen des Haarfärbemittels, wie Kräuter und Pflanzen. Insbesondere farbgebende Komponenten/ Pigmente können so ihre Eigenschaften vollständig entfalten. Ein weiterer Vorteil ist darin zu sehen, dass Pigmente und weitere Inhaltsstoffe besser zusammengehalten werden. Durch eine Art Klebeeffekt haften die Inhaltsstoffe und die Pigmente optimal an der Schuppenschicht der Haare und können so dort gezielt wirken und ihre volle Leistungsfähigkeit entfalten. Das erfindungsgemäße Haarfärbemittel deckt hierdurch besser und auch wird die Haltbarkeit des Farbergebnisses wird optimiert. Zusätzlich wird die Cremigkeit des Farbauftrags erhöht. Die Konsistenz des Farbauftrags verändert sich zusätzlich sehr positiv, eine cremige und anhaftende Textur entsteht. Dies vereinfacht das Auftragen der Haarfarbe, ermöglicht eine sparsame Anwendung, eine gleichmäßige Verteilung und begünstigt auf diese Weise ein gleichmäßiges Färbeergebnis der Haare. Durch die wasserbindenden Eigenschaften des Zuckers, behält die Haarfarbe in der Anwendung ihren kompletten Feuchtigkeitsgehalt, der Färbekomfort steigt hierdurch signifikant, da ein Abscheiden des Wassers und Verschmutzen, beispielsweise von Textilien nicht erfolgt. Darüber hinaus wird die benötigte Einwirkzeit wird durch die sehr guten Textureigenschaften der gefärbten Haare deutlich reduziert.

Zucker bezeichnen erfindungsgemäß Monosaccharide, insbesondere Pentosen, wie Ribose, und Hexosen, wie Glucose (Traubenzucker) und Fructose (Fruchtzucker), sowie Oligosaccharide, in denen 2 bis 6 Monosaccharide acetalartig miteinander verbunden sind, bevorzugt Disaccharide, insbesondere Saccharose (Rohrzucker), Maltose (Malzzucker), Lactose (Milchzucker), Trisaccharide, insbesondere Raffinose, und Tetra-, Penta- und Hexasaccharide.

Ganz besonders bevorzugt enthält der Zucker Glucose und/oder Saccharose, insbesondere Saccharose.

Durch die Körnung des Zuckers können die Eigenschaften des Haarfärbemittels ggf. weiter gesteigert und gezielt eingestellt werden. Vorzugsweise weist der Zucker eine Körnung, gemessen als Durchmesser, von höchstens 2,0 mm, bevorzugt von höchstens 1,0 mm, ganz besonders bevorzugt von höchstens 0,5 mm, insbesondere von höchstens 0,2 mm, auf.

Darüber hinaus umfasst das erfindungsgemäße Haarfärbemittel, vorzugsweise bezogen auf 100 Gewichtsteile Haarfärbemittel, 1,0 bis 10,0 Gewichtsteile, bevorzugt 2,0 bis 9,0 Gewichtsteile, ganz besonders 3,0 bis 7,0 Gewichtsteile, insbesondere 4,0 bis 5,0 Gewichtsteile, mindestens eines Liposoms.

Durch die genannte Menge des mindestens einen Liposoms wird eine weiche, cremige, seidige Konsistenz der Farbmischung und ein hoher Pflegegrad gewährleistet, welches wiederum der Austrocknung der Haare vorbeugt und einen möglichen Fettsäuremangel der Haare ausgleicht. Im Ergebnis macht dies das Haar zusätzlich geschmeidig glänzend. Gleichzeitig verhindert die genannten Mengen des mindestens einen Liposoms eine Abschwächung der Farbkonzentration und die Entstehung einer unerwünschten anhaftenden Konsistenz am Haar. Auch werden strähnig, fettige, übersättigte, schwere Haare vermieden, was das Farbergebnis wiederum negativ beeinflusst könnte.

Ein Liposom ist ein Bläschen (Vesikel), das eine wässrige Phase einschließt und dessen Membranhülle aus einer Doppelschicht von Molekülen (Doppellipidschicht) besteht. Diese Moleküle sind amphiphil, das heißt sie weisen sowohl einen unpolaren (lipophilen, fettliebenden), als auch einen polaren (hydrophilen, wasserliebenden) Teil auf. Meist handelt es sich bei den membranbildenden Molekülen um Substanzen aus der Stoffklasse der Lipide, wie zum Beispiel Phospholipide und Fettsäuren.

Da viele farbgebenden Komponenten aus den Pflanzenhaarfarben vor allem Gerbstoffe enthalten, ist die Gefahr der Austrocknung der Haare/Haarstruktur bei Verwendung derartiger Farbpigmente sehr hoch, weil sie die Proteinstruktur der Haare verändern. Durch den erfindungsgemäßen Zusatz des mindestens einen Liposoms, insbesondere Lecithin, wird dies verhindert, da es insbesondere bei hohen Temperaturen Fettsäuren zur Verfügung stellt, die als natürlicher Filmbildner fungieren und sich um das Haar legen könne. Dieser quasi Schutzmantel verhindert eine Austrocknung der Haare und ein Angreifen der Keratinschicht des Haares. Des Weiteren gewährleistet das mindestens eine Liposom, dass die Farbpigmente sowie weitere Inhaltsstoffe, wie Nährstoffe aus Heilkräutern, in die Haarstruktur transportiert werden und dort optimal wirken können. So fungieren Liposome als Transportsystem in die Haarstruktur hinein.

Im Rahmen der vorliegenden Erfindung werden Liposome, umfassend Phosphatidylcholin, besonders bevorzugt. Dabei handelt es sich bevorzugt um mindestens ein Phospholipid, das mindestens einen Ester aus Fettsäure, Glycerin, Phosphorsäure und Cholin umfasst.

Der Gewichtsanteil des mindestens einen Phosphatidylcholins, bezogen auf das Gesamtgewicht des mindestens einen Liposoms, beträgt vorzugsweise mindestens 50,0 Gewichtsprozent, bevorzugt mindestens 60,0 Gewichtsprozent, besonders bevorzugt mindestens 70,0 Gewichtsprozent, insbesondere mindestens 90,0 Gewichtsprozent.

Weiterhin kann das mindestens eine Liposom mindestens ein von Phosphatidylcholin verschiedenes Phospholipid und/oder andere Begleitstoffe enthalten. Der Anteil des mindestens einen von Phosphatidylcholin verschiedenen Phospholipids sowie der anderen Begleitstoffe, bezogen auf das Gesamtgewicht des mindestens einen Liposoms, beträgt jedoch bevorzugt höchstens 40 Gewichtsprozent, besonders bevorzugt höchstens 25,0 Gewichtsprozent, insbesondere mindestens höchstens 10,0 Gewichtsprozent.

Schließlich umfasst das erfindungsgemäße Haarfärbemittel, vorzugsweise bezogen auf 100 Gewichtsteile Haarfärbemittel, 1,0 bis 8,0 Gewichtsteile, bevorzugt 2,0 bis 7,0 Gewichtsteile, ganz besonders 3,0 bis 5,5 Gewichtsteile, insbesondere 3,5 bis 4,0 Gewichtsteile, einer Polysacharid-enthaltenden Zusammensetzung.

Durch die genannte Menge der Polysacharid-enthaltenden Zusammensetzung wird insbesondere sichergestellt, dass der Effekt des mindestens einen Farbpigments nicht abgeschwächt wird. Weiterhin wird eine negativ anhaftende verhärtete Konsistenz am Haar vermieden, die die Haare strähnig, lackig, schwer usw. wirken lassen. Auch wird sichergestellt, dass die Farbe optimal angenommen wird.

Die Polysacharid-enthaltende Zusammensetzung weist vorzugsweise wasserlösliche, filmbildende und emulgierende Eigenschaften auf und ermöglicht zweckmäßigerweise die Verbindung der Stoffe miteinander. Weiterhin verfügt die Polysacharid-enthaltende Zusammensetzung vorzugsweise über sehr gute Eigenschaften hinsichtlich der Entnetzbarkeit. Insbesondere Kräuter mit geringer Menge an Schleimstoffen, die schlecht mit anderen Stoffen binden, aber z.B. sehr gut geeignete Pigmente zum Färben besitzen, können hierdurch agglomeriert werden.

Die Polysacharid-enthaltende Zusammensetzung trocknet vorzugsweise in Verbindung mit Sauerstoff an und bildet so eine Art Überlack. Hierdurch entsteht eine Art Okklusiveffekt, der die noch bessere Versiegelung der Haarfarbe im Haar ermöglicht. Im Ergebnis kann eine faserverstärkende und stabilisierende Wirkung auf die Haarstruktur beobachtet werden. Weiterhin fungiert die Polysacharid-enthaltende Zusammensetzung aufquellend, das Haarvolumen vergrößert sich in Verbindung mit Wasser, was wiederum ein sparsames Arbeiten ermöglicht.

Zweckmäßigerweise enthält die Polysacharid-enthaltende Zusammensetzung mindestens ein Erdalkalimetall- oder Alkalimetallsalz der Arabinsäure. Darüber hinaus umfasst das Polysaccharid der Polysacharid-enthaltenden Zusammensetzung vorzugsweise Einheiten der Arabinose, der Galactose, der Rhamnose und/oder der Glucuronsäure, bevorzugt der Arabinose, der Galactose, der Rhamnose und der Glucuronsäure, insbesondere in einem Verhältnis von 3:3:1:1. Im Rahmen einer ganz besonders bevorzugten Ausführungsform der vorliegenden Erfindung besteht das Polysaccharid der Polysacharid-enthaltenden Zusammensetzung aus Einheiten der Arabinose, der Galactose, der Rhamnose und der Glucuronsäure im Verhältnis 3:3:1:1.

Weiterhin umfasst die Polysacharid-enthaltende Zusammensetzung vorzugsweise mindestens ein Glykoprotein. Bevorzugte Glykoproteine sind Makromoleküle, die aus einem Protein und einer oder mehreren kovalent gebundenen Kohlenhydratgruppen (Zuckergruppen) bestehen. Die Kohlenhydratgruppen werden gewöhnlich als posttranslationale Modifikation an Asparagin-, Serin-, Threonin- oder Hydroxylysin-Resten kovalent gebunden. Dieser Vorgang wird üblicherweise als Glykosylierung (Anlagerung von Zuckern) bezeichnet. Die gebundenen Kohlenhydratreste können stark in ihrer Größe variieren und reichen von Monosacchariden über Di- und Oligosaccharide bis zu Polysacchariden. Der Kohlenhydratanteil in Glykoproteinen kann wenige Prozent (Ribonukleasen, Thyreoglobulin) bis zu 85 Prozent (Blutgruppenantigene) betragen.

Für die Zwecke der vorliegenden Erfindung hat sich die Verwendung von Gummi arabicum, insbesondere von Gummi arabicum der Klasse 1 *(Senegalia senegal),* vorzugsweise bestehend aus großen, runden oder wurmförmigen Stücken; vorzugsweise weißlich/blass- oder bräunlich-gelb und hart; Gummi arabicum der Klasse 2 *(Senegalia senegal* und *Vachellia seyal* sowie andere *Acacia, Vachellia*), vorzugsweise bestehend aus abgerundeten, wurmförmigen oder verzweigten Stücken; vorzugsweise eher bröcklig; vorzugsweise kleiner und im Allgemeinen dunkler als Gummi arabicum der Klasse 1; und/oder von Gummi arabicum der Klasse 3, vorzugsweise umfassend andere Arten als Beimischung; vorzugsweise in Form kleiner brauner Körner, als Polysacharid-enthaltende Zusammensetzung ganz besonders bewährt, wobei die besten Ergebnisse mit Gummi arabicum der Klasse 1 oder 2, insbesondere mit Gummi arabicum der Klasse 1, erzielt werden.

Ganz besonders vorteilhaft ist die Verwendung von Gummi arabicum, insbesondere von Gummi arabicum Typ 4912, welches vorzugsweise sprühgetrocknet ist und/oder als Pulver vorliegt. Dieses wirkt vorzugsweise filmbildend auf die Haare und/oder Haut und verbindet zweckmäßigerweise Stoffe auf molekularer Ebene miteinander. Geeigneterweise verfügt es über sehr gute Eigenschaften im Hinblick auf die Einsetzbarkeit in Kräuterhaarfarben. Kräuter mit geringer Menge an Schleimstoffen, die mit anderen Stoffen schlecht binden, aber beispielweise sehr gut geeignete Pigmente zum Färben besitzen, können hierdurch agglomeriert werden. Die Komponenten trocknen in Verbindung mit Sauerstoff an, bilden so eine Art Überlack. Hierdurch entsteht eine Art Okklusiveffekt und die Farbe wird noch besser im Haar versiegelt. Darüber hinaus ist eine faserverstärkende und stabilisierende Wirkung auf die Haarstruktur zu beobachten und eine aufquellende Wirkung festzustellen, infolge der das Haarvolumen sich in Verbindung mit Wasser vergrößert, welches wiederum ein Wasser-sparsames Arbeiten ermöglicht.

Das erfindungsgemäße Haarfärbemittel kann ggf. noch weitere Inhaltsstoffe umfassen. Besonders vorteilhaft ist der Zusatz von Antioxidantien, Haarpflegemitteln, Netzmitteln, Pufferstoffen, Verdickungsmitteln, Konservierungsmitteln, Parfümölen sowie von Reduktionsmitteln zur Stabilisierung der farbgebenden Komponenten und zur Verminderung der Oxidationsempfindlichkeit der Farbstoffe.

Der Anteil der weiterer Inhaltsstoffe im erfindungsgemäßen Haarfärbemittel beträgt, vorzugsweise bezogen auf 100 Gewichtsteile Haarfärbemittel, bis zu 20,0 Gewichtsteile, bevorzugt bis zu 15,0 Gewichtsteile, insbesondere 0,1 bis 10,0 Gewichtsteile.

Vorzugsweise umfasst das erfindungsgemäße Haarfärbemittel, zweckmäßigerweise bezogen auf 100 Gewichtsteile Haarfärbemittel, 0,1 bis 4,0 Gewichtsteile, bevorzugt 0,3 bis 2,5 Gewichtsteile insbesondere 0,5 bis 1,5 Gewichtsteile Tremella Fuciformis, vorzugsweise als Pulver.

Vorzugsweise umfasst das erfindungsgemäße Haarfärbemittel, zweckmäßigerweise bezogen auf 100 Gewichtsteile Haarfärbemittel, 0,001 bis 1,0 Gewichtsteile, bevorzugt 0,01 bis 0,5 Gewichtsteile insbesondere 0,05 bis 0,2 Gewichtsteile Keraguard.

Vorzugsweise umfasst das erfindungsgemäße Haarfärbemittel, zweckmäßigerweise bezogen auf 100 Gewichtsteile Haarfärbemittel, 0,001 bis 1,0 Gewichtsteile, bevorzugt 0,01 bis 0,5 Gewichtsteile insbesondere 0,05 bis 0,2 Gewichtsteile Heilmorr-Extrakt.

Vorzugsweise umfasst das erfindungsgemäße Haarfärbemittel, zweckmäßigerweise bezogen auf 100 Gewichtsteile Haarfärbemittel, 0,1 bis 1,5 Gewichtsteile, bevorzugt 0,5 bis 1,0 Gewichtsteile insbesondere 0,65 bis 0,85 Gewichtsteile Baobab.

Vorzugsweise umfasst das erfindungsgemäße Haarfärbemittel, zweckmäßigerweise bezogen auf 100 Gewichtsteile Haarfärbemittel, 0,1 bis 1,5 Gewichtsteile, bevorzugt 0,6 bis 1,1 Gewichtsteile insbesondere 0,75 bis 0,95 Gewichtsteile bevorzugt zerkleinertes Macawurzel.

Vorzugsweise umfasst das erfindungsgemäße Haarfärbemittel, zweckmäßigerweise bezogen auf 100 Gewichtsteile Haarfärbemittel, 0,1 bis 1,5 Gewichtsteile, bevorzugt 0,5 bis 1,0 Gewichtsteile insbesondere 0,65 bis 0,85 Gewichtsteile Hanfprotein.

Vorzugsweise umfasst das erfindungsgemäße Haarfärbemittel, zweckmäßigerweise bezogen auf 100 Gewichtsteile Haarfärbemittel, 0,001 bis 1,0 Gewichtsteile, bevorzugt 0,01 bis 0,5 Gewichtsteile insbesondere 0,015 bis 0,035 Gewichtsteile bevorzugt zerkleinertes Jiaogulankraut.

Vorzugsweise umfasst das erfindungsgemäße Haarfärbemittel, zweckmäßigerweise bezogen auf 100 Gewichtsteile Haarfärbemittel, 0,1 bis 1,5 Gewichtsteile, bevorzugt 0,5 bis 1,0 Gewichtsteile insbesondere 0,65 bis 0,85 Gewichtsteile Aloe Vera.

Vorzugsweise umfasst das erfindungsgemäße Haarfärbemittel, zweckmäßigerweise bezogen auf 100 Gewichtsteile Haarfärbemittel, 0,001 bis 1,0 Gewichtsteile, bevorzugt 0,01 bis 0,5 Gewichtsteile insbesondere 0,015 bis 0,035 Gewichtsteile bevorzugt zerkleinerte Moringablätter.

Vorzugsweise umfasst das erfindungsgemäße Haarfärbemittel, zweckmäßigerweise bezogen auf 100 Gewichtsteile Haarfärbemittel, 0,001 bis 1,0 Gewichtsteile, bevorzugt 0,01 bis 0,5 Gewichtsteile insbesondere 0,015 bis 0,035 Gewichtsteile bevorzugt zerkleinertes Helmkraut.

Vorzugsweise umfasst das erfindungsgemäße Haarfärbemittel, zweckmäßigerweise bezogen auf 100 Gewichtsteile Haarfärbemittel, 0,01 bis 1,0 Gewichtsteile, bevorzugt 0,1 bis 0,5 Gewichtsteile insbesondere 0,15 bis 0,35 Gewichtsteile bevorzugt zerkleinerte Hagebuttenschale.

Vorzugsweise umfasst das erfindungsgemäße Haarfärbemittel, zweckmäßigerweise bezogen auf 100 Gewichtsteile Haarfärbemittel, 0,01 bis 1,0 Gewichtsteile, bevorzugt 0,1 bis 0,5 Gewichtsteile insbesondere 0,15 bis 0,35 Gewichtsteile bevorzugt zerkleinerte Eibischwurzel.

Vorzugsweise umfasst das erfindungsgemäße Haarfärbemittel, zweckmäßigerweise bezogen auf 100 Gewichtsteile Haarfärbemittel, 0,1 bis 1,5 Gewichtsteile, bevorzugt 0,5 bis 1,0 Gewichtsteile insbesondere 0,65 bis 0,85 Gewichtsteile bevorzugt zerkleinerte Zitronenschale.

Für die Anwendung wird das erfindungsgemäße Haarfärbemittel vorzugsweise unter Verwendung geeigneter Lösungsmittel, wie Ethanol und Wasser, insbesondere Wasser, verdünnt.

Besonders bewährt haben sich Haarfärbemittel, die, bezogen auf 100,0 Gewichtsteile Haarfärbemittel,
5,0 bis 75,0 Gewichtsteile mindestens eines Pigments,
5,0 bis 35,0 Gewichtsteile mindestens eines Zuckers,
1,0 bis 10,0 Gewichtsteile mindestens eines Liposoms,
1,0 bis 8,0 Gewichtsteile einer Polysacharid-enthaltenden Zusammensetzung bis zu 5,0 Gewichtsteile Ethanol,
sowie mindestens 20,0 Gewichtsteile Wasser umfassen.

Die Herstellung des erfindungsgemäßen Haarfärbemittels kann auf an sich bekannte Weise, beispielsweise durch Mischen der Komponenten, erfolgen. Besonders bewährt hat sich jedoch eine Vorgehensweise, bei welcher man, vorzugsweise jeweils bezogen auf 100 Gewichtsteile Haarfärbemittel,
1,0 bis 90,0 Gewichtsteile mindestens einer Zusammensetzung, die mindestens ein natürliches Pigment umfasst.,
5,0 bis 35,0 Gewichtsteile mindestens eines Zuckers,
1,0 bis 10,0 Gewichtsteile mindestens eines Liposoms,
1,0 bis 8,0 Gewichtsteile einer Polysacharid-enthaltenden Zusammensetzung sowie vorzugsweise mindestens 20,0 Gewichtsteile Wasser mischt und die Mischung auf mindestens 60°C, bevorzugt mindestens 75°C, ganz besonders bevorzugt mindestens 70°C, insbesondere mindestens 75°C, erwärmt.

Die Auftragung des erfindungsgemäßen Haarfärbemittels kann auf an sich bekannte Weise erfolgen. Zweckmäßigerweise wird das Haarfärbemittel auf die gewünschte Dosierung verdünnt und dann mit einer Spitze, einem Kamm oder einem Pinsel aufgetragen. Alternativ kann die Farbe auch in einer Farbschale gemischt werden. Die Spitze, der Kamm oder der Färbepinsel sollte zweckmäßigerweise aus Kunststoff sein, da andere Materialien, insbesondere Metall, mit dem Haarfärbemittel reagieren könnten.

Die Einwirkzeit des erfindungsgemäßen Haarfärbemittels kann grundsätzlich frei gewählt und auf den gewünschten Farbeffekt abgestimmt werden. Besonders bewährt haben sich Einwirkzeiten im Bereich von 1 Minute bis zu 45 Minuten, bevorzugt im Bereich von 5 Minuten bis 35 Minuten, insbesondere im Bereich von 15 Minuten bis 25 Minuten.

Nachfolgend wird die vorliegende Erfindung durch Beispiele und Vergleichsbeispiele weiter veranschaulicht, ohne dass hierdurch eine Einschränkung des Erfindungsgedankens auf diese Beispiele erfolgen soll.

### Testsystem:

Testfärbung von Echthaar
Rezepturmenge: 100,0 g
Temperatur zur Aufbringung des Haarfärbemittels: 75°C
Einwirkzeit: 20 Minuten
Wassermenge: 200,0 ml

### Untersuchte Haarfärbemittel:

### Erfindungsgemäßes Haarfärbemittel

| Komponente | Gewichtsteile |
|---|---|
| Zucker- Glucose | 25,000 |
| Walnussschalen | 25,000 |
| Henna | 23,000 |
| Walnussblätter | 8,000 |
| Lecithin | 5,000 |
| Akazienkollagen Senegal Gum | 4,000 |
| Faulbaumrinde zum Färben gemahlen, 10 € je kg, 52004 | 2,000 |
| Tremella Fuciformis Pulver | 1,000 |
| Kurkumawurzel | 1,000 |
| Sennesblätter | 1,000 |
| Keraguard | 0,100 |
| HEILMOOR EXTRACT | 0,100 |
| Baobab | 0,720 |
| Macawurzel | 0,864 |
| Hanfprotein | 0,720 |
| Jiaogulankraut | 0,024 |
| Aloe Vera | 0,720 |
| Moringablätter | 0,024 |
| Helmkraut | 0,024 |
| Hagebuttenschale | 0,264 |
| Eibischwurzel | 0,240 |
| Rohrzuckerpulver | 0,480 |
| Zitronenschale | 0,720 |

### Vergleichsbeispiel

| Komponente | Gewichtsteile |
|---|---|
| Walnussschalen | 36,000 |
| Henna | 34,000 |
| Walnussblätter | 20,000 |
| Faulbaumrinde zum Färben gemahlen, 10 € je kg, 52004 | 2,000 |
| Tremella Fuciformis Pulver | 1,000 |
| Kurkumawurzel | 1,000 |
| Sennesblätter | 1,000 |
| Keraguard | 0,100 |
| HEILMOOR EXTRACT | 0,100 |
| Baobab | 0,720 |
| Macawurzel | 0,864 |
| Hanfprotein | 0,720 |
| Jiaogulankraut | 0,024 |
| Aloe Vera | 0,720 |
| Moringablätter | 0,024 |
| Helmkraut | 0,024 |
| Hagebuttenschale | 0,264 |
| Eibischwurzel | 0,240 |
| Rohrzuckerpulver | 0,480 |
| Zitronenschale | 0,720 |

Die Ergebnisse der Haarfärbungen werden in Tabelle 1 zusammengefasst, wobei die Bewertung der Ergebnisse jeweils nach dem folgenden Schema erfolgte: 1 = sehr gut; 2 = gut; 3 = befriedigend; 4 = ausreichend; 5 = mangelhaft; 6 = ungenügend. Dabei wurden diese Ergebnisse wie folgt ermittelt:
∘ Konsistenz der Textur/ Streichfähigkeit
   Die Konsistenz der Textur und die Streichfähigkeit der gefärbten Haare wurde beurteilt.
∘ Verarbeitung der fertigen Textur
   Die Verarbeitung der fertigen Textur wurde bewertet.
∘ gleichmäßiges Färbeergebnis
   Die Gleichmäßigkeit des Färbeergebnis wurde bewertet.
∘ Deckkraft der Farbe
   Die Deckkraft der Farbe wurde untersucht.
∘ Grauhaarabdeckung
   Die durch die Haarfärbungen erzielbare Grauhaarabdeckung wurde bewertet.
∘ Wasserabsonderung aus Textur heraus
   Die Absonderung von Wasser aus der Textur heraus wurde untersucht.
∘ Glanz- und Pflegewirkung
   Die Glanz- und Pflegewirkung wurde bewertet.
∘ Volumen
   Das durch die Haarfärbung erzielbare Volumen wurde bewertet.
∘ Haarstruktur
   Die Struktur der gefärbten Haare wurde visuell bewertet.
∘ Auswaschen - Entfernung aus dem Haar
   Es wurde die Auswaschbarkeit der Haarfärbung mit 10 l Wasser (25°C) untersucht.
∘ Wasserverbrauch
   Es wurde die benötigte Wassermenge zum Auswaschen der Haarfärbung untersucht.

**Tabelle 1**

| | Vergleichsbeispiel | Erfindungsgemäßes Haarfärbemittel |
|---|---|---|
| Konsistenz der Textur/ Streichfähigkeit | 4 | 1 |
| Verarbeitung der fertigen Textur | 4 | 1 |
| gleichmäßiges Färbeergebnis | 3 | 1 |
| Deckkraft der Farbe | 3 | 1 |
| Grauhaarabdeckung | 4 | 1 |
| Wasserabsonderung aus Textur heraus | 3 | 1 |
| Glanz und Pflegewirkung | 3 | 1 |
| Volumen | 3 | 2 |
| Haarstruktur | 3 | 1 |
| Auswaschen - Entfernung aus dem Haar | 4 | 2 |
| Wasserverbrauch | 5 | 2 |

## Patentansprüche

1. Haarfärbemittel, umfassend
1,0 bis 90,0 Gewichtsteile mindestens einer Zusammensetzung, die mindestens ein natürliches Pigment umfasst,
5,0 bis 35,0 Gewichtsteile mindestens eines Zuckers,
1,0 bis 10,0 Gewichtsteile mindestens eines Liposoms,
1,0 bis 8,0 Gewichtsteile einer Polysacharid-enthaltenden Zusammensetzung.

2. Haarfärbemittel nach Anspruch 1, **dadurch gekennzeichnet, dass** das natürliche Pigment Henna rot, Henna neutral, Henna schwarz, Kamillenblüte, Sandelholz, schwarzem Tee, grünem Tee, Faulbaumrinde, Salbei, Blauholz, Krappwurzel, Catechu, Sedre, Alkannawurzel, Kurkumawurzel, Walnussschale, Walnussblätter, Sennesblätter, Indigo und/oder Cochenille umfasst.

3. Haarfärbemittel nach Anspruch 2, **dadurch gekennzeichnet, dass** das natürliche Pigment Henna, Kurkumawurzel, Walnussschale, Walnussblätter, Sennesblätter und/oder Faulbaumrinde umfasst.'

4. Haarfärbemittel nach Anspruch 2, **dadurch gekennzeichnet, dass** das Haarfärbemittel
∘ 10,0 bis 40,0 Gewichtsteile vorzugweise zerkleinerte Walnussschalen;
∘ 8,0 bis 38,0 Gewichtsteile Gewichtsteile Henna;
o 1,0 bis 20,0 Gewichtsteile vorzugweise zerkleinerte Walnussblätter und/oder;
∘ 0,1 bis 10,0 Gewichtsteile vorzugweise zerkleinerte Faulbaumrinde umfasst.

5. Haarfärbemittel nach Anspruch 1 oder 2, **dadurch gekennzeichnet, dass** der Zucker Glucose oder Saccharose enthält.

6. Haarfärbemittel nach mindestens einem der vorgehenden Ansprüche, **dadurch gekennzeichnet, dass** der Zucker eine Körnung, gemessen als Durchmesser, von höchstens 2,0 mm, bevorzugt von höchstens 1,0 mm, ganz besonders bevorzugt von höchstens 0,5 mm, insbesondere von höchstens 0,2 mm, aufweist.

7. Haarfärbemittel nach mindestens einem der vorgehenden Ansprüche, **dadurch gekennzeichnet, dass** das Liposom mindestens ein Phosphatidylcholin umfasst.

8. Haarfärbemittel nach mindestens einem der vorgehenden Ansprüche, **dadurch gekennzeichnet, dass** das Haarfärbemittel Lecithin umfasst.

9. Haarfärbemittel nach mindestens einem der vorgehenden Ansprüche, **dadurch gekennzeichnet, dass** die Polysacharid-enthaltende Zusammensetzung mindestens ein Erdalkalimetall- oder Alkalimetallsalz der Arabinsäure umfasst.

10. Haarfärbemittel nach mindestens einem der vorgehenden Ansprüche, **dadurch gekennzeichnet, dass** das Polysaccharid der Polysacharid-enthaltenden Zusammensetzung Einheiten der Arabinose, der Galactose, der Rhamnose und/oder der Glucuronsäure umfasst.

11. Haarfärbemittel nach mindestens einem der vorgehenden Ansprüche, **dadurch gekennzeichnet, dass** die Polysacharid-enthaltende Zusammensetzung mindestens ein Glykoprotein umfasst.

12. Haarfärbemittel nach mindestens einem der vorgehenden Ansprüche, **dadurch gekennzeichnet, dass** die Polysacharid-enthaltende Zusammensetzung Gummi arabicum umfasst.

13. Haarfärbemittel nach Anspruch 12, **dadurch gekennzeichnet, dass** die Polysacharid-enthaltende Zusammensetzung Gummi arabicum der Klasse 1 umfasst.

14. Haarfärbemittel nach mindestens einem der vorgehenden Ansprüche, **dadurch gekennzeichnet, dass** das Haarfärbemittel weiterhin mindestens ein Antioxidans, mindestens ein Haarpflegemittel, mindestens ein Netzmittel, mindestens einen Pufferstoff, mindestens ein Verdickungsmittel, mindestens ein Konservierungsmittel, mindestens ein Parfümöl und/oder mindestens ein Reduktionsmittel zur Stabilisierung mindestens einer farbgebenden Komponente und/oder zur Verminderung der Oxidationsempfindlichkeit des Farbstoffs enthält.

15. Verfahren zur Herstellung eines Haarfärbemittels nach mindestens einem der vorangehenden Ansprüche, **dadurch gekennzeichnet, dass** man
1,0 bis 90,0 Gewichtsteile mindestens einer Zusammensetzung, die mindestens ein natürliches Pigment umfasst,
5,0 bis 35,0 Gewichtsteile mindestens eines Zuckers,
1,0 bis 10,0 Gewichtsteile mindestens eines Liposoms,
1,0 bis 8,0 Gewichtsteile einer Polysacharid-enthaltenden Zusammensetzung Mischt und die Mischung auf mindestens 60°C, bevorzugt mindestens 75°C, ganz besonders bevorzugt mindestens 70°C, insbesondere mindestens 75°C, erwärmt.

## Claims

1. A hair dye comprising
1.0 to 90.0 parts by weight of at least one composition comprising at least one natural pigment
5.0 to 35.0 parts by weight of at least one sugar,
1.0 to 10.0 parts by weight of at least one liposome,
1.0 to 8.0 parts by weight of a polysaccharide-containing composition.

2. The hair dye according to claim 1, **characterised in that** the natural pigment comprises henna red, henna neutral, henna black, chamomile flower, sandalwood, black tea, green tea, alder buckthorn bark, sage, logwood, madder root, catechu, sedre, alkanna root, turmeric root, walnut shell, walnut leaves, senna leaves, indigo and/or cochineal.

3. The hair dye according to claim 2, **characterised in that** the natural pigment comprises henna, turmeric root, walnut shell, walnut leaves, senna leaves and/or alder buckthorn bark.

4. The hair dye according to claim 2, **characterised in that** the hair dye comprises
∘ 10.0 to 40.0 parts by weight of preferably comminuted walnut shell;
∘ 8.0 to 38.0 parts by weight of henna;
∘ 1.0 to 20.0 parts by weight of preferably comminuted walnut leaves and/or;
∘ 0.1 to 10.0 parts by weight of preferably comminuted alder buckthorn bark.

5. The hair dye according to claim 1 or 2, **characterised in that** the sugar contains glucose or sucrose.

6. The hair dye according to at least one of the preceding claims, **characterised in that** the sugar has a grain size, measured as diameter, of at most 2.0 mm, preferably of at most 1.0 mm, very particularly preferably of at most 0.5 mm, in particular of at most 0.2 mmm.

7. The hair dye according to at least one of the preceding claims, **characterised in that** the liposome comprises at least one phosphatidylcholine.

8. The hair dye according to at least one of the preceding claims, **characterised in that** the hair dye comprises lecithin.

9. The hair dye according to at least one of the preceding claims, **characterised in that** the polysaccharide-containing composition comprises at least one alkaline earth metal salt or alkali metal salt of arabic acid.

10. The hair dye according to at least one of the preceding claims, **characterised in that** the polysaccharide of the polysaccharide-containing composition comprises units of arabinose, galactose, rhamnose and/or glucuronic acid.

11. The hair dye according to at least one of the preceding claims, **characterised in that** the polysaccharide-containing composition comprises at least one glycoprotein.

12. The hair dye according to at least one of the preceding claims, **characterised in that** the polysaccharide-containing composition comprises gum arable.

13. The hair dye according to claim 12, **characterised in that** the polysaccharide-containing composition comprises class 1 gum arable.

14. The hair dye according to at least one of the preceding claims, **characterised in that** the hair dye furthermore contains at least one antioxidant, at least one haircare agent, at least one wetting agent, at least one buffer substance, at least one thickener, at least one preservative, at least one perfume oil and/or at least one reducing agent to stabilise at least one colouring component and/or to reduce the oxidation sensitivity of the colourant.

15. A method for producing a hair dye according to at least one of the preceding claims, **characterised in that**
1.0 to 90.0 parts by weight of at least one composition comprising at least one natural pigment,
5.0 to 35.0 parts by weight of at least one sugar,
1.0 to 10.0 parts by weight of at least one liposome, and
1.0 to 8.0 parts by weight of a polysaccharide-containing composition
are mixed and the mixture is heated to at least 60°C, preferably at least 75°C, very particularly preferably at least 70°C, in particular at least 75°C.

## Revendications

1. Colorant pour cheveux comprenant
1,0 à 90,0 parties en poids d'au moins une composition qui comprend au moins un pigment naturel,
5,0 à 35,0 parties en poids d'au moins un sucre,
1,0 à 10,0 parties en poids d'au moins un liposome,
1,0 à 8,0 parties en poids d'une composition contenant un polysaccharide.

2. Colorant pour cheveux selon la revendication 1, **caractérisé en ce que** le pigment naturel comprend du henné rouge, du henné neutre, du henné noir, de la fleur de camomille, du bois de santal, du thé noir, du thé vert, de l'écorce de bourdaine, de la sauge, du bois de campêche, de la racine de garance, du catéchou, du jujubier sauvage, de la racine d'orcanette des teinturiers, de la racine de curcuma, de la coque de noix, des feuilles de noyer, des feuilles de séné, de l'indigo et/ou de la cochenille.

3. Colorant pour cheveux selon la revendication 2, **caractérisé en ce que** le pigment naturel comprend du henné, de la racine de curcuma, de la coque de noix, des feuilles de noyer, des feuilles de séné et/ou de l'écorce de bourdaine.

4. Colorant pour cheveux selon la revendication 2, **caractérisé en ce que** le colorant pour cheveux comprend
∘ 10,0 à 40,0 parties en poids de coques de noix de préférence réduites ;
∘ 8,0 à 38,0 parties en poids de henné ;
∘ 1,0 à 20,0 parties en poids de feuilles de noyer de préférence réduites et/ou ;
∘ 0,1 à 10,0 parties en poids d'écorce de bourdaine de préférence réduite.

5. Colorant pour cheveux selon la revendication 1 ou la revendication 2, **caractérisé en ce que** le sucre contient du glucose ou du saccharose.

6. Colorant pour cheveux selon au moins une des revendications précédentes, **caractérisé en ce que** le sucre présente une granulométrie, mesurée sous la forme d'un diamètre, d'au maximum 2,0 mm, de préférence d'au maximum 1,0 mm, de manière particulièrement préférée d'au maximum 0,5 mm, notamment d'au maximum 0,2 mm.

7. Colorant pour cheveux selon au moins une des revendications précédentes, **caractérisé en ce que** le liposome comprend au moins une phosphatidylcholine.

8. Colorant pour cheveux selon au moins une des revendications précédentes, **caractérisé en ce que** le colorant pour cheveux comprend de la lécithine.

9. Colorant pour cheveux selon au moins une des revendications précédentes, **caractérisé en ce que** la composition contenant un polysaccharide comprend au moins un sel de métal alcalino-terreux ou un sel de métal alcalin de l'acide arabique.

10. Colorant pour cheveux selon au moins une des revendications précédentes, **caractérisé en ce que** le polysaccharide de la composition contenant un polysaccharide comprend des motifs d'arabinose, de galactose, de rhamnose et/ou d'acide glucuronique.

11. Colorant pour cheveux selon au moins une des revendications précédentes, **caractérisé en ce que** la composition contenant un polysaccharide comprend au moins une glycoprotéine.

12. Colorant pour cheveux selon au moins une des revendications précédentes, **caractérisé en ce que** la composition contenant un polysaccharide comprend de la gomme arabique.

13. Colorant pour cheveux selon la revendication 12, **caractérisé en ce que** la composition contenant un polysaccharide comprend de la gomme arabique de classe 1.

14. Colorant pour cheveux selon au moins une des revendications précédentes, **caractérisé en ce que** le colorant pour cheveux contient en outre au moins un antioxydant, au moins un agent de soin pour cheveux, au moins un agent mouillant, au moins une substance tampon, au moins un épaississant, au moins un conservateur, au moins une huile de parfum et/ou au moins un agent réducteur permettant la stabilisation d'au moins un composant colorant et/ou permettant la réduction de la sensibilité à l'oxydation du colorant.

15. Procédé de fabrication d'un colorant pour cheveux selon au moins une des revendications précédentes, **caractérisé en ce que**
1,0 à 90,0 parties en poids d'au moins une composition qui comprend au moins un pigment naturel,
5,0 à 35,0 parties en poids d'au moins un sucre,
1,0 à 10,0 parties en poids d'au moins un liposome, et
1,0 à 8,0 parties en poids d'une composition contenant un polysaccharide sont mélangées et que le mélange est chauffé jusqu'à au moins 60 °C, de préférence
au moins 75 °C, de manière particulièrement préférée au moins 70 °C, notamment au moins 75 °C.
